# EUROPEAN PATENT SPECIFICATION

(11) **EP 1 965 739 B1**
(45) Date of publication and mention of the grant of the patent: **19.06.2013**
(21) Application number: 05822009.6
(22) Date of filing: 16.12.2005
(51) Int. Cl.: A61F 13/15, A61F 13/511, A61F 13/539, A61F 13/537

(54) **ABSORBENT ARTICLE AND METHOD FOR MANUFACTURING AN ABSORBENT ARTICLE**
SAUGFÄHIGER ARTIKEL UND VERFAHREN ZUR HERSTELLUNG EINES SAUGFÄHIGEN ARTIKELS
ARTICLE ABSORBANT ET PROCEDE DE FABRICATION D'UN ARTICLE ABSORBANT

(43) Date of publication of application: 10.09.2008
(73) Proprietor: SCA Hygiene Products AB, 405 03 Göteborg (SE)
(72) Inventor: HÅKANSSON, Carin, S-427 38 Billdal (SE); WAHLSTRÖM, Johan, S-560 27 Tenhult (SE)
(74) Representative: Andersson, Per Rune
(86) International application number: PCT/SE2005/001967
(87) International publication number: WO 2007/069967

(56) References cited:
- WO-A1-01/03626
- US-A- 5 681 300
- US-A1- 2005 136 224

## Description

### TECHNICAL FIELD:

The present invention relates to an absorbent product such as an incontinence pad, a sanitary towel, a panty liner or the like, comprising a liquid-permeable top layer, a preferably liquid-impermeable bottom layer and an absorbent core arranged between the top layer and the bottom layer.

The invention also relates to a method for the manufacture of an absorbent product such as an incontinence pad, a sanitary towel, a panty liner or the like, in conjunction with which the product is executed with a liquid-permeable top layer, a preferably liquid-impermeable bottom layer and an absorbent core arranged between the top layer and the bottom layer.

### BACKGROUND ART:

In conjunction with the manufacture of absorbent products such as sanitary towels, panty liners and incontinence pads, processes relating to the joining together of materials of various kinds are usually performed. For example, this may involve the manufacture of a liquid-permeable surface layer, which can consist of a laminate with a nonwoven layer comprising synthetic or natural fibres, which must be joined together with a wadding material. An absorbent core is usually arranged under this laminate. A liquid-proof bottom layer is then arranged under the absorbent core in a previously disclosed manner.

Depending on what type of absorbent product is to be manufactured, the various layers of material can be treated in various ways. For example, the upper nonwoven layer may be perforated. The absorbent core can also be provided, if required, with a superabsorbent material.

In conjunction with the manufacture of the absorbent product, it is also possible, in accordance with the prior art, to glue the surface layer and the bottom layer together. This means that the absorbent core and the wadding material are then placed between the surface layer and the bottom layer before this gluing process is carried out.

Ultrasound technology is used in addition in certain processes that are arranged for the processing of absorbent products, for example in conjunction with the joining together of two or more layers of material. This technology is also previously disclosed and is suitable, for example, for joining together the type of relatively thin material layers that are used in absorbent products. In the case of such joining together, which is also referred to as ultrasonic welding, a laminate can be formed by the actual layers of material.

In conjunction with the manufacture of an absorbent product, a particular requirement may be to join together a perforated nonwoven layer with a wadding material so that a laminate of the aforementioned kind is formed. A process of this kind can then be performed by means of ultrasonic welding, that is to say without gluing. In order to complete the product, the absorbent core must then be placed between the above-mentioned surface laminate and the bottom layer, after which the bottom layer is glued securely to the surface laminate.

Disclosed in patent document WO 99/49825 is a material laminate which comprises a first material layer of a nonwoven material and a second layer of material which consists of a wadding material. These two layers of material are joined together by means of ultrasonic welding.

WO01/03626 discloses an absorbent article comprising a material laminate comprising a first fluid pervious fibrous material layer and a second fluid pervious porous material layer wherein at least one of the material layers comprises a thermoplastic material. The layers are connected together by bond sites within the thermoplastic material. The bonding may be carried out by ultrasonic bonding. The laminate comprises a bond pattern in the central portion, in which the spot shaped bonds are at a greater distance from each other than the bonds in the side portions.

US 5,681,300 discloses an absorbent article in which a top sheet and an acquisition layer are fused together by means of heat or pressure bonds. The bonds are distributed over the entire body surface of the main body portion of the sanitary napkin. The main body is inboard of any liquid impervious seam, such as a seam around the periphery of the sanitary napkin.

In those cases in which the absorbent core and the wadding material are of a size that is significantly smaller than that of the surface layer and the bottom layer, an absorbent product is then formed with a core that is relatively rigid, but in which the parts of the surface layer and the bottom layer that are situated to the side of the rigid core can be perceived as weak and loose. A further problem associated with such a product is that the seal along the edges of the surface laminate is not always perfect unless a gluing operation is carried out in this area. On the whole, the finished product may be perceived as less attractive by the wearer. Against the background of the foregoing, it can be established that the need exists for improved absorbent products in which the above-mentioned problems can be eliminated.

### DISCLOSURE OF INVENTION:

A principal object of the present invention is thus to solve the above-mentioned problems and to make available an improved absorbent product and an improved method for the manufacture of absorbent products.

The above object is achieved with a product of the kind referred to by way of introduction, in which the top layer is joined together with a subjacent admission material by means of ultrasonic processing, and the laminate that is defined by the top layer and the admission material when these are joined together includes a reinforcement executed by ultrasonic processing essentially along its peripheral edge.

The object is also achieved by means of a method of the kind referred to by way of introduction, which comprises: the joining together of the top layer and a subjacent admission material by means of ultrasonic processing, and the forming by means of ultrasonic processing of a reinforcement running essentially along the peripheral edge of the laminate that is defined by the top layer and the admission material when these are joined together.

Certain advantages are achieved through the invention. Firstly, it can be noted that an attractive product is obtained without the weak and loose edges, that is to say in the area outside the absorbent core. In addition, a highly effective edge seal of the laminates is achieved, which are defined by the top layer and the admission material when these are joined together, where the risk of any leakage and spread of liquid via their peripheral edge can be minimized or eliminated. The invention is also capable, in the case in which the joining together of the top layer and the admission material and the sealing of the edges takes place in a single stage, of providing major advantages of a process engineering nature, where in particular the welding pattern for the admission material and the edge seal is "synchronized" in an optimal fashion. In other words, the finished product will exhibit a high degree of concordance with regard to the pattern of welding for the lamination and the pattern of welding for the edge seal. This in turn provides additional advantages in that the surface welding pattern and the edge seal can be designed as a continuous pattern, which not least may be visually appealing. The invention also provides simpler contour cutting of the finished product without any loose fibres.

The invention also provides an advantage in relation to the prior art, in which any seal between a surface material and an admission material - which can be executed by means of a gluing procedure in a previously disclosed fashion - can be less effective in respect of the admission characteristics of the product.

It can be stated as a general rule that the invention provides advantages from a combined process and design perspective, because it can be used to form welded patterns and embossed areas that are both technically optimal with regard to, for example, strength, and visually attractive. At the same time, the invention permits a very effective manufacturing process in view of the ability to perform two process stages simultaneously.

### BRIEF DESCRIPTION OF DRAWINGS:

The invention is described below in conjunction with a preferred illustrative embodiment and the accompanying drawings, in which
- Figure 1: is a perspective view of an absorbent product in accordance with the present invention in a separated state;
- Figure 2: is a perspective view of the absorbent product in a partially assembled state; and
- Figure 3: shows a view from above of the aforementioned product in the completed state.

### MODE(S) FOR CARRYING OUT THE INVENTION:

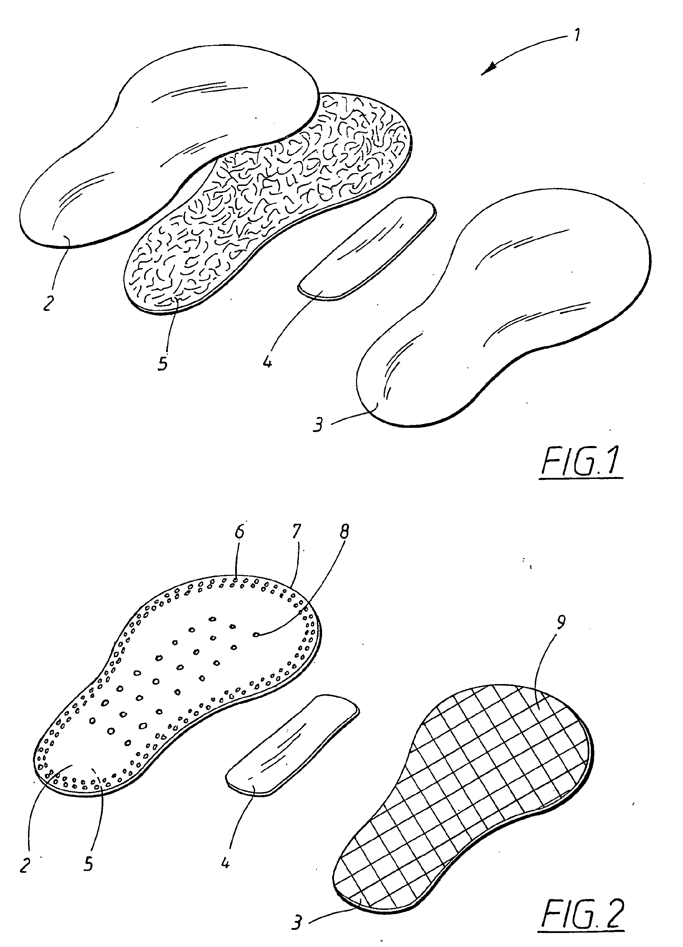

Figure 1 is a perspective view of an absorbent product 1 in accordance with the present invention, viewed in its separated state. The product 1 comprises a first covering layer in the form of a liquid-permeable top layer 2, which is arranged on the side of the product 1 which, during use, is intended to face towards the wearer. The product 1 also comprises a second covering layer in the form of a liquid-impermeable bottom layer 3, which is arranged on the side of the product 1 which, during use, is intended to face away from the wearer. The product 1 also comprises an absorbent core 4 arranged between the top layer 2 and the bottom layer 3.

The absorbent core 4 is appropriately manufactured from a suitable fibre material in the form of natural or synthetic fibres with absorbent characteristics, or a mixture or natural fibres and synthetic fibres or other absorbent materials of a previously disclosed kind that are suitable for use in, for example, sanitary towels, incontinence pads and panty liners. The absorbent core 4 can also comprise a predetermined proportion, for example 40-60%, of superabsorbent material, that is to say polymer materials in the form of particles, fibres, flakes or the like, which possess the ability to absorb and chemically bind liquid equivalent to several times their own weight to form an aqueous gel. This imparts a very high liquid-absorbent capacity to the finished product 1.

It must also be noted that the absorbent core 4 can exhibit different forms, for example an essentially elongated and rectangular form, as can be appreciated from Figure 1, or alternatively an even more irregular form. The absorbent core 4 also preferably has rounded edges.

The liquid-permeable top layer 2 in accordance with Figure 1 preferably consists of a fibrous material, for example a soft nonwoven material, although alternatively it can consist of other materials or material laminates. The top layer 2 is preferably fully or partially perforated, although alternatively it can be entirely imperforate. The top layer 2 can appropriately consist of a perforated plastic film, for example a thermoplastic plastic material such as polyethylene or polypropylene, or a mesh-like layer of synthetic or textile material. Synthetic fibres, such as polyethylene, polypropylene, polyester, nylon or the like are preferably used as nonwoven material. Mixtures of different types of fibres can also be used for the aforementioned nonwoven material. The invention is not, however, restricted in principle to use only for top layers which consist of nonwoven material, but can also be applied in conjunction with the processing of other materials, for example films of thermoplastics such as polyethylene or polypropylene.

The invention can also be implemented with a top layer which consists of different types of laminates or combinations of laminates. For example, the top layer may consist of only a single laminate extending along the entire surface of the product 1, or alternatively of a plurality of different laminates which cover parts of the product 1. In the event that the product 1 consists of a plurality of laminates, for example divided up into a plurality of longitudinal parts having different laminate sections, these different laminate sections can consist of different materials and can have different characteristics. For example, each laminate section can then have different types of perforation, hole positioning, dimensions, hydrophobicity, etc. The different laminate sections can then be joined together by means of ultrasonic welding in a previously disclosed fashion that is not described here in greater detail.

The liquid-permeable top layer is preferably manufactured from a material that exhibits characteristics such as dryness and softness during the time when the absorbent product 1 is being worn, because this top layer is in contact with the wearer's body. It is also desirable for the top layer to have a soft and textile-like surface which remains dry, even in the event of repeated wetting. The top layer can consist of a nonwoven material, for example, with a soft and smooth surface, such as a spunbond material made from polypropylene fibres. A perforated, hydrophobic nonwoven material may be used in order to permit the surface that is closest to the wearer's body to be kept dry, in conjunction with which openings are formed in the material that are larger than the holes between the fibres in the material. In this way, liquid can be led down through the perforated openings in the top layer to the subjacent absorbent core. Other examples of materials for the top layer are perforated plastic films such as a perforated polyester film. The top layer can be joined together with the subjacent bottom layer and the absorbent core, for example by means of adhesive, ultrasonic jointing or by means of some form of thermal bonding. The top layer is preferably a perforated nonwoven material with an opening density of 3-15, preferably 6-12 and most preferred of all 7-9 openings/cm².

In accordance with one preferred embodiment, the top layer contains small perforations which surround the openings in the top layer for the purpose of further increasing the intake of liquid and the acquisition characteristics. The perforations in the top layer normally have a density in the range from 20-500, preferably 70-250, and most preferred of all 120-170 perforations/cm².

It is desirable for the thickness of the top layer to be as large as possible, because a large thickness has a positive effect on the acquisition. A balance is required, however, in order not to influence the softness of the material, and for this reason a thickness in the range from 1.3 to 1.7 mm, and preferably ca. 1.5 mm, has been found to be appropriate.

The top layer can also be a three-dimensional laminate of nonwoven and plastic film with a base weight of 42.5 gsm. The nonwoven material can be a carded, thermally bonded material based 100% on polypropylene, with a base weight of 18 gsm. The plastic film can be hydrophilic, pre-perforated (with small holes) and manufactured from a mixture of polyethylene and polypropylene, with a base weight of 22.5 gsm.

The nonwoven part of the top layer can also be a spunbond nonwoven material, an air-thru nonwoven material, a spunlace nonwoven (hydroentangled) material, a meltblown nonwoven material, or a combination of these. The raw material can be polypropylene (PP), polyethylene (PE) polyester (PET), polyamide (PA), or a combination of these. If a combination is used, this can be a mixture of fibres from different polymers, although each fibre can also include different polymers (e.g. PP/PE bi-component fibres or PP/PE copolymers). Where appropriate, the plastic film can consist of PE or PP, PET, PLA or amyl (or, for that matter, any other thermoplastic polymer), or a mixture or copolymers of the aforementioned polymers.

The perforated top layer can also be manufactured from a single layer of material, such as a nonwoven material or a film (as described above).

The top layer can have an opening size of 1.6 to 3.2 mm in the direction of the machine (longitudinal) and 0.9 to 2.3 mm in the transverse direction. The opening size can be between 0.1 and 6.0 mm in diameter.

The openings in the top layer can be oval and slightly elongated in the direction of the machine. The openings can be round/circular or oval in the direction of the machine or the transverse direction.

The open surface in the top layer can lie in the interval between 2 and 60%, preferably between 5 and 30%, and particularly preferred ca. 14%.

The bottom layer 3 is preferably liquid-impermeable (or should at least possess high resistance to penetration by liquid) and is thus so arranged as to prevent any leakage of excreted fluid from the product 1. The bottom layer 3, on the other hand, may be executed so that it is vapour-permeable. For this purpose, the bottom layer 3 may be manufactured from a liquid-impermeable material which consists appropriately of a thin and liquid-proof plastic film. For example, plastic films of polyethylene, polypropylene or polyester can be used for this purpose. Alternatively, a laminate of nonwoven and plastic film or other suitable material layers can be used as a liquid-proof bottom layer. 3. In a previously disclosed fashion, the under side of the bottom layer 3 can be provided with beads of adhesive (not illustrated) or some other previously disclosed fastening means, which can then be utilized for the attachment of the product 1 to an item of clothing. The product 1 can also be provided with wings, that is to say folding flaps which, in a previously disclosed fashion, are arranged along the sides of the product 1 and can be utilized in conjunction with the attachment of the product 1.

The product 1 also includes a further layer of material in the form of an admission material 5 (also referred to as an acquisition material) in accordance with the embodiment in the form of a wadding material having an appropriately selected thickness and elasticity, which is intended to be positioned in this case between the absorbent core 4 and the top layer 2. The admission material 5 possesses essentially the same dimensions as the top layer 2, with the exception of its thickness, however, which can deviate from the thickness of the top layer 2. It is also possible to establish that the admission material 5 can consist of materials other than wadding materials. For example, it may consist of a so-called airlaid material, which is usually based on cellulose fibres. The admission material 5 can also incorporate fibrous materials in order to impart a suitable, balanced rigidity to it. The admission material 5 can also incorporate an appropriate quantity of thermoplastic fibres in order to permit ultrasonic welding.

The admission material can appropriately be a porous, elastic, relatively thick layer of material, for example in the form of a fibrous wadding material, a carded fibre wadding, a tow material, or some other kind of bulky and/or resilient fibre material with a high instantaneous liquid intake capacity that is capable temporarily of storing liquid before it is absorbed by the subjacent absorbent core. The admission material can also be in the form of a porous foam material. It can also consist of two or more layers of material. In accordance with a preferred embodiment, the admission material can extend towards the lateral edges of the product, that is to say it possesses essentially the same form as the top layer. In this way, advantages can be achieved in respect of liquid distribution, edge sealing, etc.

The product 1 is so arranged that the top layer 2 and the admission material 5 must be joined together to form a laminate in conjunction with the manufacture of the finished product 1. The principles for joining together the absorbent layer 1 will now be described, initially with reference to Figure 2. The invention is based on the fact that processing by means of ultrasound technology is utilized in conjunction with the manufacture of the product 1. Such ultrasound devices are previously disclosed and are therefore not described in detail here. Ultrasound devices can be arranged for processing by means of ultrasound technology, for example in the form of welding, embossing, cutting, perforating, or some other type of ultrasound processing. In the embodiment that will now be described below, the ultrasound processing that is utilized is of the type ultrasonic welding, that is to say the joining together of two or more layers of material.

In the embodiment of the invention described here, an ultrasound device (not illustrated) is used for the purpose of initially joining together, that is to say laminating, the top layer 2 and the admission material 5. This joined laminate consisting of the top layer 2 and the admission material 5 is indicated in schematic fashion in Figure 2. The bottom layer 3 must also be joined to the underside of the laminate, which consists of the top layer 2 and the admission material 5, in conjunction with which the absorbent core 4 must be placed in the correct position on top of the bottom layer 3. Before this is done, however, an operation is performed which represents a significant part of the present invention, namely that the laminate which has just been formed, that is to say consisting of the top layer 2 and the admission material 5, is also provided with an edge reinforcement 6, which preferably runs around the periphery 7 of the top layer 2 in accordance with what is indicated schematically with broken lines in Figure 2. This edge reinforcement 6, which also performs the function of a seal along the periphery 7 that is formed by the top layer 2 and the inlet material 5, is executed with the help of the aforementioned ultrasound device. The edge reinforcement 6 is appropriately formed as a number of discrete welded points that are manufactured in a previously disclosed fashion with the help of the aforementioned ultrasound device and extends from the periphery 7 and towards the inside of the product for a certain distance, for example in the order of 2-6 mm.

It must be noted that the edge reinforcement 6 is preferably executed at the same time as the top layer 2 and the admission material 5 are laminated together. In other words, the ultrasound device is used preferably in such a way that the lamination of the top layer 2 and the admission material 5 takes place in the same process stage as the forming of the edge reinforcement 6. On the one hand, a welded pattern 8 which preferably extends over at least that part of the top layer 2 of the product 1 that is intended to cover the absorbent core 4 and, on the other hand, the welded pattern 6 which runs along the peripheral edge 7 of the top layer 2, are thus generated in this process. The edge reinforcement 6 is also utilized during the process as a seal in the form of an ultrasonic weld which bonds together fibres in the admission material 5. This provides the invention with an advantage, in that the material in these areas is more rigid.

The invention can be utilized during processes for the manufacture of absorbent products, both when the admission material 5 consists of a material that is broader than the final form of the product 1 (which accordingly requires that the laminate consisting of the top layer and the admission material must be contour-cut along the lamination), and when the admission material has the same form as the top layer (which is equivalent to the top layer and the admission material having been formed in advance to essentially the same outer form).

The edge reinforcement 6, that is to say the pattern of welded points that is formed along the peripheral edge 7 of the product 1, can be uniform insofar as the same width and characteristics in the welded joint in other respects are applicable around the whole of the periphery 7. Alternatively, different parts of the periphery 7 can be given different types of welded joints, for example a more widely diffused welded joint at certain points around the periphery 7 than at other points around the periphery 7. Similarly, for example, different welded patterns and welding depths can be applied around different sections of the periphery 7.

The invention is not restricted to the execution of the lamination of the top layer 2 and the admission material 5 in a single process stage, but can also be implemented in such a way that this takes place in the form of two or more consecutive process stages.

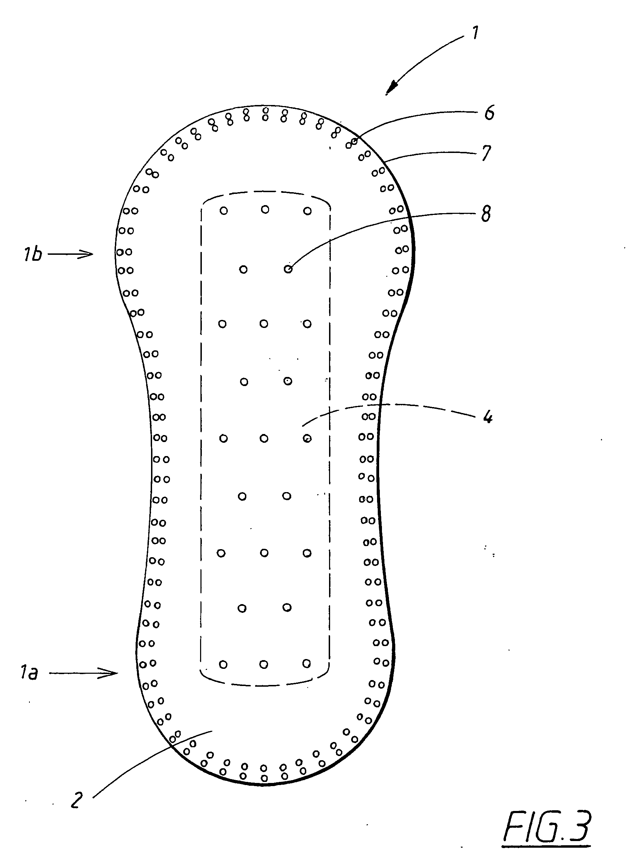

As a final stage in the manufacture of the product 1, the bottom layer 3 is glued to the top layer 2 and the admission material 5, in conjunction with which a layer of adhesive 9 is first applied to the top side of the bottom layer 3. The product 1 as a whole is then pressed together, in conjunction with which precautions are taken to ensure that the absorbent core 4 has been positioned in the correct way. Figure 3 shows a finished product 1 that has been manufactured in accordance with the underlying principles of the present invention. It can be appreciated from Figure 3 that the bottom layer 3 has then been joined together with the laminate, which in turn comprises the top layer 2 and the admission material 5, in conjunction with which the absorbent core 4 is positioned between the admission material 5 and the bottom layer 3. Other joining techniques can be used as an alternative instead of the above-mentioned adhesive procedure, for example different types of mechanical jointing.

The product 1 is specially designed in accordance with this embodiment for use as an incontinence pad, although, as mentioned above, the principles of the invention are not restricted to only this type of product. For example, the invention is also suitable for use in the manufacture of products in the form of sanitary towels and panty liners. As far as the physical design of the product 1 in accordance with the invention is concerned, it can be stated that it preferably exhibits a form that is asymmetrical in relation to an imaginary centre line that runs across the longitudinal direction of the product 1. This is equivalent to the central part of the product 1 having a smaller width than its rear part 1 a (see Figure 3) and its front part 1b, and to its rear part 1 a having a smaller width than its front part 1 b.

This asymmetrical form can be clearly appreciated from Figure 3. Alternatively, the product 1 in accordance with the invention can have a form resembling an hourglass, that is to say with a smaller width in its central area than in its front and rear parts respectively, or an essentially T-shaped form. The product 1 can also be given a physical form that is adapted to the use of a thong by the wearer.

The invention offers advantages in particular in the case of an asymmetrical form, in the sense that the edge along the front part 1b of the product 1 is given reinforcing characteristics, since the front part 1 b in fact has a width that exceeds the width of the rear part 1 a and can exhibit a tendency to become loose and weak. This can lead in turn to the disadvantages mentioned by way of introduction.

As far as the dimensions of the product 1 are concerned, it is also true that a relatively small absorbent product usually has a length of less than 22 cm and a width of these than 8.5 cm. The invention is not restricted to any specific dimensions, however, but can be adapted with regard to its dimensioning to the application in question. In one appropriate embodiment as a panty liner, the length of the product 1 is in the order of 13-20 cm, and its width (at its central part) is in the order of 4-6 cm. The product 1 can have larger dimensions, for example, in an embodiment as an incontinence pad.

In summary, the invention is based on the formation of a surface laminate by joining together the perforated nonwoven layer which forms the top layer 2 and the admission material 5 by means of ultrasonic welding. This surface laminate is also provided with an edge reinforcement 6. It must be noted in this case that the top layer 2 can consist of one or more layers of material and must be capable of being welded by means of ultrasonic welding. This in turn calls for a certain minimum quantity of thermoplastic material in order to permit the welding procedure. The bottom layer 3 is then glued in place, in conjunction with which the absorbent core 4 is positioned in the correct manner.

As mentioned above, the laminate that is formed by the top layer 2 and the admission material 5 must include thermoplastic material in order to permit ultrasonic welding. Furthermore, the welding of the laminate that is formed by the top layer 2 and the admission material 5 offers an advantage in the sense that the risk of unsealed edge parts and delamination can be minimized.

The invention is not restricted to what is indicated above, but different embodiments are possible within the scope of the Patent Claims. For example, the invention is not restricted to being utilized with any particular type of absorbent product. Moreover, the invention can also be realized with many types of material and material combinations. For example, the top layer 2 can consist of nonwoven material or a film material.

## Claims

1. Absorbent product (1), such as an incontinence pad, a sanitary towel, a panty liner or the like, comprising a liquid-permeable top layer (2), a preferably liquid-impermeable bottom layer (3) and an absorbent core (4) arranged between the top layer (2) and the bottom layer (3), the top layer (2) being joined together with a subjacent admission material (5) by means of ultrasonic processing to form a laminate, which laminate includes a thermoplastic material, ***characterized in that*** the laminate that is defined by the top layer (2) and the admission material (5) when these are joined together includes a reinforcement (6) executed with ultrasonic processing essentially along its peripheral edge (7), forming an edge seal of the laminate so that the risk of any leakage and spread of liquid via the peripheral edge (7) is minimized or eliminated, and wherein the reinforcement (6) extends from the periphery (7) and towards the inside of the product for a distance of 2-6 mm.

2. Absorbent product (1) according to Claim 1, ***characterized in that*** the top layer (2) consists at least partially of a perforated nonwoven material.

3. Absorbent product (1) according to Claims 1 or 2, ***characterized in that*** the aforementioned absorbent core (4) comprises a superabsorbent material.

4. Absorbent product (1) according to one or other of Claims 1-3, ***characterized in that*** the aforementioned admission material (5) consists of a wadding material.

5. Absorbent product (1) according to one or other of Claims 1-4, ***characterized in that*** it is designed with an asymmetrical, or alternatively an essentially hourglass-shaped contour.

## Patentansprüche

1. Saugfähiges Produkt (1), wie z.B. eine Inkontinenzeinlage, eine Binde, eine Slipeinlage oder dergleichen, umfassend eine flüssigkeitsdurchlässige Oberschicht (2), eine vorzugsweise flüssigkeitsundurchlässige Unterschicht (3) und einen saugfähigen Kern (4), der zwischen der Oberschicht (2) und der Unterschicht (3) angeordnet ist, wobei die Oberschicht (2) mit einem unterliegenden Aufnahmematerial (5) mittels Ultraschallbehandlung zusammengefügt ist, um ein Laminat zu bilden, welches Laminat thermoplastisches Material umfasst,
**dadurch gekennzeichnet, dass**
das Laminat, das durch die Oberschicht (2) und das Aufnahmematerial (5), wenn diese miteinander verbunden sind, definiert ist, eine Verstärkung (6) umfasst, die durch Ultraschallbehandlung im Wesentlichen entlang ihrer Außenkante (7) ausgeführt ist und eine Kantenabdichtung des Laminats bildet, so dass die Gefahr einer Ausströmung und Ausbreitung von Flüssigkeit durch die Außenkante (7) minimiert oder beseitigt wird, und wobei sich die Verstärkung (6) vom Umfang (7) und gegen das Innere des Produkts über einen Abstand von 2-6 mm erstreckt.

2. Saugfähiges Produkt (1) nach Anspruch 1, **dadurch gekennzeichnet, dass** die Oberschicht (2) zumindest teilweise aus einem perforierten Vliesmaterial besteht.

3. Saugfähiges Produkt (1) nach Anspruch 1 oder 2, **dadurch gekennzeichnet, dass** der obenerwähnte saugfähige Kern (4) ein supersaugfähiges Material umfasst.

4. Saugfähiges Produkt (1) nach irgendeinem der Ansprüche 1-3, **dadurch gekennzeichnet, dass** das obenerwähnte Aufnahmematerial (5) aus einem Füllmaterial besteht.

5. Saugfähiges Produkt (1) nach irgendeinem der Ansprüche 1-4, **dadurch gekennzeichnet, dass** es mit einer asymmetrischen oder alternativ einer im Wesentlichen stundenglasförmigen Kontur ausgeformt ist.

## Revendications

1. Produit absorbant (1), tel qu'une protection contre l'incontinence, une serviette hygiénique, un protège-slip ou un objet similaire, comprenant une couche supérieure perméable aux liquides (2), une couche inférieure de préférence imperméable aux liquides (3) et un noyau absorbant (4) disposé entre la couche supérieure (2) et la couche inférieure (3), la couche supérieure (2) étant assemblée avec un matériau sous-jacent d'admission (5) au moyen d'un traitement par ultrasons pour former un stratifié, ledit stratifié comprenant une matière thermoplastique, **caractérisé en ce que** le stratifié qui est défini par la couche supérieure (2) et le matériau d'admission (5) lorsque ceux-ci sont reliés l'un à l'autre comprend un renforcement (6) réalisé avec un traitement par ultrasons essentiellement le long de son bord périphérique (7), formant un bord d'étanchéité du stratifié si bien que le risque de toute fuite ou dispersion de liquide via le bord périphérique (7) est minimisé ou éliminé, et dans lequel le renforcement (6) s'étend à partir du bord périphérique (7) et vers l'intérieur du produit pour une distance de 2 à 6 mm.

2. Produit absorbent (1) selon la revendication 1, **caractérisé en ce que** la couche supérieure (2) est constituée au moins partiellement d'un matériau non-tissé perforé.

3. Produit absorbent (1) selon la revendication 1 ou 2, **caractérisé en ce que** ledit noyau absorbant (4) comprend un matériau superabsorbant.

4. Produit absorbent (1) selon l'une quelconque des revendications 1-3, **caractérisé en ce que** ledit matériau d'admission (5) est constitué d'un matériau de rembourrage.

5. Produit absorbent (1) selon l'une quelconque des revendications 1-4, **caractérisé en ce qu**'il est conçu avec un contour asymétrique ou alternativement essentiellement en forme de sablier.
